# EUROPEAN PATENT APPLICATION

(11) **EP 3 933 395 A1**
(43) Date of publication of application: **05.01.2022**
(21) Application number: 20762607.8
(22) Date of filing: 28.02.2020
(51) Int. Cl.: G01N 27/403, C12Q 1/26

(54) **REUSABLE, PORTABLE, WIRELESS BIOSENSOR**

(30) Priority: 28.02.2019 ES 201930187
(71) Applicant: Consejo Superior De Investigaciones Científicas, 28006 Madrid (ES)
(72) Inventor: DEL CAMPO GARCÍA, Francisco Javier, 08193 Cerdanyola del Vallès (Barcelona) (ES); GUIMERÁ BRUNET, Antón, 08193 Cerdanyola del Vallès (Barcelona) (ES); ALLER PELLITERO, Miguel, 08193 Cerdanyola del Vallès (Barcelona) (ES); DEI, Michele, 08193 Cerdanyola del Vallès (Barcelona (ES)
(74) Representative: Pons
(86) International application number: PCT/ES2020/070144
(87) International publication number: WO 2020/174117

(57) **Abstract**

The present invention relates to a biosensor device for the determination of analytes, which is non-invasive by means of displaying a colour change, and starting from a sample of liquid. The present invention falls within the field of devices for health, control when playing sports, occupational safety and the food industry.

## Description

The present invention relates to a biosensor device for the non-invasive determination of analytes, by means of displaying a colour change, and starting from a liquid sample. The present invention falls within the field of devices for health, control when playing sports, occupational safety and the food industry.

### BACKGROUND OF THE INVENTION

At present, in the field of portable chemical sensors and, more specifically, in the field of sweat measurements, we find that one of the trends consists of developing skin patches. These sensors of a chemical nature have two distinct portions, the portion with the sensor and the portion with the control instrumentation. These portions generally have very different useful lives; while the control electronics can last for years without noticeable loss of performance, the sensor portion deteriorates with use to a greater or lesser extent depending on the working conditions thereof, in some cases enabling only one measurement to be performed.

Because of this, current systems are designed such that the instrumentation can be reused, while making the portion of the sensor low-cost and disposable. The paradigmatic example is that of personal glucometers, wherein the sensor is located on a disposable plastic strip, the cost of which is a few cents of a euro. This strip is physically connected to the instrumentation when it is inserted into the reader. Moreover, each strip enables only one measurement, and requires a drop of blood to be drawn by means of a finger prick, which is uncomfortable and can lead to infections.

In order to improve the quality of life of patients suffering from diabetes, the industry seeks to develop non-invasive systems which enable the measurement of glycaemia in other body fluids such as sweat. In this sense, the Abbott company has recently put on the market a device which enables the semi-continuous measurement of blood glucose in a minimally invasive manner (in interstitial fluid). The system consists of 2 portions: a wireless reader and a capsule with a diameter of 5cm and thickness of 5mm which incorporates the biosensor, the control electronics thereof, a small memory, a radio frequency communication module, and a button battery which powers the assembly. This capsule is placed on the arm, under the shoulder, wherein it is fastened by means of an adhesive, while the biosensor, which is located at the end of a needle with a length of 5mm, remains inserted in the arm of the patient. The biosensor has a useful life of 2 weeks, according to the manufacturer, after which time the biosensor capsule is removed and replaced with a new one. Despite the advantages thereof, the complexity of the biosensor portion translates into a high cost of the product per biosensor capsule.

Moreover, in the state of the art it is worth noting the works of groups wherein either the portion of the sensor has to be physically connected to the instrumentation by means of cables, or the sensor portion integrates an entire electronic circuitry which enables the control thereof and the communication of data [M. Dei, J. Aymerich, M. Piotto, P. Bruschi, F. J. del Campo, and F. Serra-Graells, "CMOS Interfaces for Internet-of-Wearables Electrochemical Sensors: Trends and Challenges," Electronics, vol. 8, no. 2, p. 150, Feb. 2019.]. Furthermore, these devices prove the need to perform a continuous calibration in said devices to ensure the measurement.

That is why there is currently a need for a portable and disposable device capable of carrying out numerous measurements over time, thus capable of enormously reducing the environmental impact of this waste.

### DESCRIPTION OF THE INVENTION

The present invention provides a non-invasive biosensor device for determining analytes from a liquid sample, such as sweat, effluvia from foods, etc., which is portable and wireless and can return to the initial state thereof ready to perform a new measurement by using a pulse received without needing cables, either by means of electromagnetic waves, more preferably radio frequencies, and without needing complex electronics or instrumentation.

The advantages of this device are:
The possibility of reusing the sensor more than once, moving away from the devices of the state of the art, wherein the sensor is disposable after a single use.

It is not necessary to draw blood to deposit it on the sensor, but rather a non-invasive measurement can be performed, for example, starting from the sweat of the subject. This moves it away from the current state of the art, wherein it is performed using a device that performs a continuous measurement in an invasive manner. The lifespan of the sensor can reach two weeks.

A simplified calibration/possibility of self-calibration, with fewer steps and problems with respect to the state of the art, since the sensors of the state of the art generally require calibration at the beginning and at the end of measurements; however, in the device of the present invention, given that it has information on colour and the rate of change thereof, it is possible to correct the concentration during measurements without needing patterns. Thus, by having the information on colour at the end of the previous measurement, it is possible to know the charge contained in the sensor before each measurement. By knowing the charge and the rate of change, it is possible to calculate the analyte concentration in each measurement. In the case of using an optical reader of the measurement, said reader can perform the necessary calculations to correct any possible drops in charge that may occur in the sensor. Consequently, the device does not require external patterns.

The wireless device of the present invention does not need additional silicon components to function, and furthermore it avoids the connection of the sensors by means of cables to a measurement instrumentation. This facilitates both the construction and the use of the device. In fact, the device is designed to work with near-field radio frequency. This has the advantage that the signal does not have to be encrypted for security reasons. Furthermore, the optical or colourimetric reader which will read the sensor should ideally be very close to it in order to facilitate the reading and prevent measurement errors.

The charge and discharge currents are not affected by capacitive currents during the measurement, as it is a coulometric measurement (of charge). This can be an advantage, especially in cases wherein the analyte is found in very low concentrations. Furthermore, if the measurement were electrochemical (voltamperometric), the charge/discharge of the double layer could affect the measurement. However, as it is an optical measurement, this problem does not occur since the colour change is strictly associated with a faradaic process (oxidation/reduction).

The maximum use is of two electrodes compared to three in the state of the art. In conventional electrochemical measurements based on controlling potential of the working electrode, 3 electrodes are used. Applying these techniques with 2 electrodes means losing control of the potential of the working electrode (sensor), which could be counterproductive, because at that moment we stop knowing at which potential it is being measured, and therefore which processes are contributing to the current and which ones are not. In summary, it is rare to find amperometric sensors based on systems with only 2 electrodes because in them the auxiliary electrode must also fulfil the reference function, and the problem is that when current passes, the potential thereof is modified. This causes the potential of the sensor to fluctuate, and makes the measurement become less reliable. Therefore, from the point of view of an electrochemist, an amperometric system with 2 electrodes would not be very attractive. Moreover, in a conventional amperometric measurement system, the direct connection of the antenna to the electrodes can result in the application of alternating potential differences of the order of several volts between the 2 electrodes of the system. However, in amperometric sensors, potentials of less than 1V are generally applied against the corresponding reference electrode. In fact, it seeks to apply the lowest possible potentials which guarantee a purely diffusive control of the measured current. With this, we seek to prevent interference from other electroactive molecules capable of reacting in the electrode in that range of potentials. Furthermore, in some cases, the application of extreme working potentials can lead to irreversible damage to the sensor. In the case of Prussian Blue (PB), if an excessively high oxidation potential is applied, "Berlin Green" is produced in a semi-reversible process. This green form of the complex is unstable and reduces the useful life of the blue form. Moreover, if an excessive reduction potential is applied, in the absence of K⁺ ions in the medium, the structure of the material can be damaged.

Problems associated with the lack of control of the electrochemical potential of the sensor are prevented, since the measurement performed is not electrochemical but optical (colourimetric).

The device of the present invention has application in a wide range of technical fields such as health, sports, occupational safety, the environment and the food industry. Initially, the presented invention aims to develop sensors or biosensors for non-invasive measurements, preferably in sweat, by means of the use of activity monitors, smart watches, and other wearable devices, although it would also enable the manufacture of "smart labels" for food quality control.

The first aspect of the present invention is a wireless and portable biosensor device for displaying a colour change from a first colour to a second colour, wherein said second colour indicates the presence of an analyte in a liquid sample, and the first colour indicates the absence of the analyte or a non-reading state, the device being characterised in that it comprises a stratified structure comprising:
- a transparent substrate comprising a face a and a face b, optionally the substrate comprises at least two holes connecting the two faces, a and b, of the substrate;
- a conductive structure configured as the main structure of an antenna and at least one conductive track of the working and auxiliary electrodes, comprising conductive silver paste;
- at least two transparent conductive structures configured to function at least as two transducers, one for the working electrode and the other for the auxiliary electrode;
- at least one electrochromic structure, configured to function at least as a working electrode;
- a transparent structure configured to deposit the sample on it and comprising
   ▪ chitosan; and
   ▪ an oxidase enzyme.
- a main dielectric structure which is configured to insulate the conductive tracks from the conductive structure;
- optionally a second dielectric structure located on the conductive structure and/or on the electrochromic one, leaving at least one area of said electrochromic layer exposed, and wherein said dielectric structure layer comprises at least two holes;
- at least one conductive track configured to connect with the conductive structure in order to close the circuit; and
- a diode;

wherein the device functions in direct current; wherein the antenna is configured so that, with a radio frequency between 10.56 MHz and 16.56 MHz, the measurement of the device is restarted until the non-reading state or state of absence of analyte in the sample; and wherein the device is configured to be reset to the non-reading state or state of absence of analyte thereof when receiving a signal of a certain radio frequency between 10.56 MHz and 16.56 MHz.

In the present invention, "substrate" or "transparent substrate" is understood as any transparent material with a thickness between 10 µm and 250 µm, preferably between 30 µm and 100 µm, more preferably selected from: polyethylene terephthalate (PET) and derivatives, polyurethane, polystyrene and derivatives, polyvinyl alcohol and derivatives, and cellulosic materials. In the present invention, optionally the substrate comprises at least two holes or pathways.

In the present invention, "holes or pathways" are understood as the perforated areas with at least two small holes (60-120 µm in diameter) which enable the conductive tracks to be connected in order to close the circuit, preferably through the substrate so that the silver paste may pass from one face of said substrate to the other, forming an electrical contact between both faces, or through the second dielectric structure.

In the present invention "dielectric structure" or "dielectric" are understood as all insulating materials or bad conductors which are used to protect the conductive tracks and prevent leakage currents or the oxidation of the tracks when they come in contact with the liquid sample wherein the analyte is found for the measurement thereof. In the present invention, it preferably relates to any photocurable resin, thermosetting resin and resin curable by drying or chemical reaction, more preferably it is a photocurable resin.

In the present invention, a "transducer" is understood as a component of an electrode capable of transforming or converting a certain manifestation of input energy into another different one at the output, but with very small values in relative terms with respect to a generator. The transparent conductive structure configured to function as a transducer is formed by a transparent conductive material which can be selected from at least one layer of PEDOT:PSS; at least one thin layer with a size between 3 nm and 100 nm of a material selected from Au, Pt, graphene, carbon nanotubes, fullerenes; at least one layer comprising tin oxide; and any combination of the above; and preferably is PEDOT:PSS. In the present invention, the device comprises at least two transparent conductive structures configured to function as transducers, one for the working electrode and the other for the auxiliary electrode. In the absence of a conductive or electrochromic structure on said transducer of the auxiliary electrode, said transducer will act as an auxiliary electrode.

In the present invention, "electrochromic structure" is understood as a material which, in reaction to an electric current, changes colour. This structure is configured to function as a working electrode. In the present invention, each electrochromic structure comprises a pigment, preferably Prussian Blue. More preferably it comprises:
a. microparticles, selected from mixed indium and tin oxide (ITO), of the core-shell type wherein the core is SiO₂ and wherein the shell is a mixed antimony and tin oxide, and a combination of the foregoing; and
b. an ink comprising
   i. a pigment, preferably Prussian Blue; and
   ii. a binding resin, preferably a Viton solution; and
wherein the microparticle:ink ratio is 2.5:1;
and wherein the microparticles and the ink form a homogeneous particulate mixture with a particle size of said mixture between 3 µm and 12 µm.

For the elaboration of the electrochromic structure, a modification of the pigment, for example Prussian blue, can be performed by means of a growth of said pigment on microparticles, thus generating an ink usable for the electrochromic structure of the device of the present invention. In order to perform this growth of the pigment on the microparticles, it is performed in a slightly acidic aqueous medium containing iron and ferrous or ferrocyanide salts. Subsequently, once the pigment has grown on the particles, they are filtered, washed, dried and ground in order to obtain the finest powder possible.

This obtained powder is added to a binding resin in the form of a solution, all in a polar solvent, such as, for example and not limited to, 2-Butoxyethylacetate. In this manner, a much greater contact between the pigment and the rest of the components is achieved, for which reason the electrical losses are reduced, as well as the voltage necessary for using the device of the present invention. The composition of the paste for making the electrochromic structure comprises:
microparticles plus pigment, preferably Prussian Blue, in a percentage between 15% and 60% by weight;
binding resin, preferably Viton, in a percentage between 4% and 15% by weight; and solvent in a percentage between 25% and 81% by weight.

The preferred composition of the electrochromic structure used in the present invention comprises: 20% by weight of microparticles and pigment, 8% by weight of binding resin and 72% by weight of solvent.

In the present invention, "antenna" is understood as the metal structure with a spiral shape which acts as an interface between the near electromagnetic field and the currents which are induced in the same structure. The antenna transduces the signal of the electromagnetic oscillating field into alternating currents.

In the present invention, "diode" is understood as any device capable of rectifying the alternating current of the radio frequency signal; the anode of the diode must be connected such that electrons flow from the diode to the electrochromic structure, and not the other way around. Otherwise, the system would not function as planned. Therefore, not only is the rectification of the current important, but also the polarity thereof. Furthermore, in the case that an alternating signal is applied to the reversible system comprising the pigment, the net charge flowing in the absence of said diode is zero. This means that there would be no noticeable colour change in the working electrode (electrochromic structure). In the present invention, the diode can be printed, or manufactured by means of a combination of microfabrication and printing processes, or a discrete silicon component is used. In the present invention, "Schottky diode" is understood as the diode which has a metal-N joint, in other words, wherein the main metal is doped with an electron donor semiconductor, causing a high switching speed which enables very high frequency signals to be rectified and excess current to be eliminated in high-intensity circuits.

In the present invention, "liquid sample" is understood as a sample which can be selected from a biological fluid or a food, liquid or solid, which may or may not be previously treated for the determination of the analyte containing it.

"Biological fluid" is understood in the present invention as blood, sweat, saliva, tears and urine. In a preferred embodiment, the biological fluid is sweat.

In a preferred embodiment of the wireless and portable biosensor device of the present invention, the conductive structure, comprising conductive silver paste, is located directly on the face a of the substrate. In a more preferred embodiment of the device, the transparent conductive structure, configured to function as a transducer, is located on the previous conductive structure, located on the face a of the substrate.

In an even more preferred embodiment of the device, it comprises a stratified structure comprising:
- a transparent substrate comprising a face a and a face b, and wherein the substrate comprises at least two holes connecting the two faces, a and b, of the substrate;
- a first layer of conductive structure configured as the main structure of an antenna and conductive tracks of the working and auxiliary electrodes, comprising conductive silver paste, and located on at least one portion of the face a of the substrate;
- at least two second layers of transparent conductive structure, located on the first layer of conductive structure, and configured to function at least as two transducers, one for the working electrode and the other for the auxiliary electrode;
- at least one third layer of electrochromic structure, located on at least one area of the second layer of transparent conductive structure, and configured to function at least as a working electrode;
- a fourth layer of transparent structure adhered on the third layer of electrochromic structure, and configured to deposit the sample to be determined on it and comprising:
   ▪ chitosan; and
   ▪ an oxidase enzyme.
- a fifth layer of main dielectric structure located on the previous layers leaving at least one area exposed corresponding to the deposit area of the sample of the fourth layer, and which is configured to insulate the conductive tracks of the main structure of the first layer;
- a sixth layer of a conductive track comprising conductive silver paste, and located on the face b of the substrate and configured to connect, by means of the holes in the substrate, with the conductive structure of the first layer and to close the circuit; and
- a diode on the sixth layer;

wherein the device functions in direct current;
wherein the antenna is configured so that, with a radio frequency between 10.56 MHz and 16.56 MHz, the measurement of the device is restarted until the non-reading state or state of absence of analyte in the sample; and
wherein the device is configured to be reset to the non-reading state or state of absence of analyte thereof when receiving a signal of a certain radio frequency between 10.56 MHz and 16.56 MHz.

In another preferred embodiment of the wireless and portable biosensor device of the present invention, the transparent conductive structure, configured to function as a transducer, is located directly on the substrate. In a more preferred embodiment of the device, the conductive structure comprises conductive silver paste and is located directly on the transparent conductive structure. In an even more preferred embodiment of the device, it comprises a stratified structure comprising:
- a transparent substrate comprising a face a and a face b, and wherein the substrate comprises at least two holes connecting the two faces, a and b, of the substrate;
- at least one first layer of transparent conductive structure located on the face a of the substrate and configured to function at least as two transducers, one for the working electrode and the other for the auxiliary electrode;
- a second layer of conductive structure, configured as the main structure of an antenna and conductive tracks of the working and auxiliary electrodes, and comprising conductive silver paste, and in contact with at least one portion of the first layer of transparent conductive structure;
- at least one third layer of electrochromic structure, located on at least one surface of the second layer of conductive structure, configured to function at least as a working electrode;
- a fourth layer of transparent structure adhered on the third layer of electrochromic structure configured to deposit the sample on it and comprising
   ▪ chitosan; and
   ▪ an oxidase enzyme.
- a fifth layer of main dielectric structure located on the previous layers leaving at least one area or surface exposed or uncovered corresponding to the deposit area of the sample described in the fourth layer, and which is configured to insulate the conductive tracks of the second layer;
- a sixth layer of a conductive track comprising conductive silver paste, and located on the face b of the substrate and configured to connect, by means of the holes in the substrate, with the conductive structure of the first layer and to close the circuit; and;
- a diode on the sixth layer;

wherein the device functions in direct current;
wherein the antenna is configured so that, with a radio frequency from 10.56 MHz to 16.56 MHz, the measurement of the device is restarted until the non-reading state or state of absence of analyte in the sample; and
wherein the device is configured to be reset to the non-reading state or state of absence of analyte thereof when receiving a signal of a certain radio frequency between 10.56 MHz and 16.56 MHz.

In another even more preferred embodiment of the device, it comprises a stratified structure comprising:
- a transparent substrate comprising a face a and a face b;
- at least two first layers of transparent conductive structure located on one of the faces of the substrate and configured to function at least as two transducers, one for the working electrode and the other for the auxiliary electrode;
- a second layer of conductive structure, configured as the main structure of an antenna and conductive tracks of the working and auxiliary electrodes, and comprising conductive silver paste, and in contact with at least one portion of the first layer of transparent conductive structure;

- a third layer of dielectric structure configured to insulate the conductive tracks of the second layer;
- at least one fourth layer of electrochromic structure, located on at least one area of the second layer of conductive structure, configured to function at least as a working electrode;
- a fifth layer of a second dielectric structure located on the fourth layer of electrochromic structure leaving at least one area exposed or uncovered, corresponding to the deposit area of the sample, and wherein said dielectric structure comprises at least two holes;
- a sixth layer of transparent structure located, in the area exposed or uncovered by the fifth layer, on the fourth layer of electrochromic structure, configured to deposit the sample on it and comprising:
   ▪ chitosan; and
   ▪ an oxidase enzyme.
- a seventh layer of a conductive track, configured to connect with the second layer of conductive structure through the holes in the fifth layer of dielectric structure and to close the circuit; and
- a diode on the seventh layer of conductive structure;

wherein the antenna is configured so that, with a radio frequency from 10.56 MHz to 16.56 MHz, the measurement of the device is restarted until the non-reading state or state of absence of analyte in the sample; and
wherein the device is configured to be reset to the non-reading state or state of absence of analyte thereof when receiving a signal of a certain radio frequency between 10.56 MHz and 16.56 MHz.

In another more preferred embodiment of the device of the present invention, the diode is a Schottky diode and has dimensions between 0.8 mm and 1.6 mm.

The oxidase enzyme to be used in the transparent structure of the device of the present invention will depend on the analyte to be determined in the liquid sample. For example, and without limiting ourselves, the oxidase enzyme is selected from glucose oxidase, lactose oxidase, maltose oxidase, urate oxidase or ethanol oxidase, when the analyte is selected from glucose, lactose, maltose, uric acid, or ethanol, respectively. In a preferred embodiment, the oxidase enzyme is glucose oxidase. In an even more preferred embodiment, the transparent structure comprising glucose oxidase additionally comprises the enzyme mutarotase, wherein said enzyme helps improve the sensitivity of the glucose detection by means of the device of the present invention.

The device could additionally comprise a structure on the transducer of the auxiliary electrode. Thus, in a preferred embodiment, the device additionally comprises a transparent conductive structure or an electrochromic structure, either of the two located directly on the transparent conductive layer configured as a transducer of the auxiliary electrode and which are configured to function as an auxiliary electrode.

By using a transparent conductive structure as an auxiliary electrode, the colour change is visible from the face b of the device, thus favouring the display of the measurement.

If the device of the invention comprises the additional electrochromic structure configured to function as an auxiliary electrode, this auxiliary electrode acts as a charge reserve layer to balance the reaction of the electrochrome and thus enables working with a lower voltage. By using the same electrochromic material as the material for the auxiliary electrode as the one used for the working electrode, the manufacturing of both electrodes is done in one step, and there is a lower energy cost to activate the device, since there will not be a potential difference between the working electrode and the auxiliary electrode; and there will be no extra potential difference to be applied in order to perform the measurement and reset the device.

Another preferred embodiment of the wireless and portable biosensor device of the present invention wherein the structure of the electrochrome is deposited on the auxiliary electrode or in contact with both electrodes. By means of this configuration, the working voltage of the device is reduced.

Another aspect of the invention relates to a method for obtaining the device of the present invention, characterised in that it comprises the following steps:
a. making holes in a transparent substrate comprising a face a and a face b, preferably the substrate is made of PET with a thickness between 30 µm - 100 µm, by means of a technique selected from CO₂ laser, mechanical punching and a combination of the above, such that they connect the two faces of the substrate;
b. printing a conductive structure on the face a of the substrate using conductive silver paste in order to form the spiral structure of the antenna and the conductive tracks of the working and auxiliary electrodes, making at least one printed area coincide with the holes in the step (a);
c. printing at least two transparent conductive structures on the printing performed in step (b) by using a transparent conductor, preferably PEDOT:PSS, to function as transducers, one for the working electrode and the other for the auxiliary electrode;
d. printing an electrochromic structure on a portion of the conductive structure obtained in step (c);
e. printing a conductive track on the face b of the substrate, such that the ends of the tracks coincide with the areas of pathways and make contact with the ends of the antenna and with the transducer of the auxiliary electrode;
f. depositing a dielectric structure, preferably a layer of dielectric resin, on the face a of the material obtained after step (d) leaving at least one area exposed corresponding to the deposit area of the sample of electrochromic structure;
g. depositing a diode on the conductive track of the face b of the substrate obtained in step (e), between one of the ends of the antenna and one of the electrodes making up the sensor, by means of silver paste or an anisotropic conductive adhesive; and
h. adhering a transparent structure comprising chitosan and an oxidase enzyme in the deposit area of the sample which remained exposed after depositing the dielectric structure in step (f).

In a preferred embodiment of the method for obtaining the device of the present invention described, it comprises additionally a step (c') after step (c) of printing a layer on the transducer of the auxiliary electrode selected from a transparent conductive structure or an electrochromic structure.

Another aspect of the present invention relates to an alternative method for obtaining the device of the present invention, characterised in that it comprises the following steps:
a. printing on the face a of the transparent substrate, preferably by means of screen printing, a first conductive structure, using conductive silver paste to form the conductive tracks constituting the spiral structure of the antenna and the conductive tracks of the working and auxiliary electrodes;
b. on the first conductive structure obtained in step (a), depositing at least two second transparent conductive structures using a transparent conductor to function as transducers, one for the working electrode and the other for the auxiliary electrode, preferably selected from
   - at least one layer of PEDOT: PSS;
   - at least one thin layer with a size between 3 nm and 10 nm of a material selected from Au, Pt, graphene, carbon nanotubes, fullerenes;
   - at least one layer comprising tin oxide;
   - and any combination of the above;
c. printing the third structure made of electrochromic material on the second transparent conductive structure obtained in step (b);
d. depositing a fourth dielectric structure, preferably a layer of dielectric resin, on the face a of the material obtained after step (c) leaving at least one area exposed corresponding to the deposit area of the sample of the third structure;
e. printing at least one conductive track on the structure made of dielectric material deposited in step (d), such that the ends of the tracks coincide with the inner ends of the antenna and with the contact of the auxiliary electrode deposited in step (a);
f. depositing a new structure made of dielectric material, defining the working areas of the electrodes, the contacts for the diode and other possible electronic or measurement components, and the area wherein the sample to be analysed will be confined.
g. assembling a diode on the conductive tracks in the area suitable to do so by means of silver paste or an anisotropic conductive adhesive; and
h. adhering a transparent structure comprising chitosan and an oxidase enzyme in the deposit area of the sample of the structure free from dielectric structure of step (f).

In a preferred embodiment of the method for obtaining the device of the present invention described, it comprises
additionally a step (c') after step (c) of printing a layer on the transducer of the auxiliary electrode selected from a transparent conductive structure or an electrochromic structure.

Another aspect of the invention relates to another alternative method for obtaining the device of the present invention, characterised in that it comprises the following steps
a. making holes in a transparent substrate comprising a face a and a face b, preferably the substrate is made of PET with a thickness between 30-100µm, by means of a technique selected from CO₂ laser, mechanical punching and a combination of the above, such that they connect the two faces of the substrate;
b. printing on the face a of the substrate at least two transparent conductive structures by using a transparent conductor in order to function as transducers, one for the working electrode and the other for the auxiliary electrode, preferably selected from
   µ at least one layer of PEDOT: PSS;
   µ at least one thin layer with a size between 3 nm and 100 nm of a material selected from Au, Pt, graphene, carbon nanotubes, fullerenes;
   µ at least one layer comprising tin oxide;
   µ and any combination of the above;
c. printing by means of screen printing a second conductive structure on the printing performed in step (b) by using conductive silver paste to form the conductive tracks constituting the spiral structure of the antenna and the conductive tracks of the working and auxiliary electrodes, making at least one printed area coincide with the holes of step (a);
d. printing the electrochromic material on the second conductive structure obtained in step (c);
e. printing a series of conductive tracks on the second face b of the substrate, such that the ends of the tracks coincide with the areas of pathways and make contact with the ends of the antenna and with the contact of the transducer of the auxiliary electrode;
f. depositing a fifth dielectric structure, preferably a layer of dielectric resin, on the face a of the material obtained after step (e) leaving at least one area exposed corresponding to the deposit area of the sample of fourth electrochromic structure;
g. assembling a diode on the conductive tracks of face b of the substrate obtained in step (e), in the area suitable to do so by means of silver paste or an anisotropic conductive adhesive; and
h. adhering a transparent structure comprising chitosan and an oxidase enzyme in the deposit area of the sample of the structure free from dielectric structure of step (f).

In a preferred embodiment of the method for obtaining the device of the present invention described, it comprises
additionally a step (d') after step (d) of printing a layer on the transducer of the auxiliary electrode selected from a transparent conductive structure or an electrochromic structure.

Another aspect of the present invention relates to another alternative method for obtaining the device of the present invention, characterised in that it comprises the following steps
a. printing on the face a of the transparent substrate, preferably by means of screen printing, a first conductive structure, using conductive silver paste to form the conductive tracks constituting the spiral structure of the antenna and the conductive tracks of the working and auxiliary electrodes;
b. printing on the first conductive structure obtained in step (a) at least two second transparent conductive structures by using a transparent conductor to function as transducers, one for the working electrode and the other for the auxiliary electrode, preferably selected from
   - at least one layer of PEDOT: PSS;
   - at least one thin layer with a size between 3 nm and 10 nm of a material selected from Au, Pt, graphene, carbon nanotubes, fullerenes;
   - at least one layer comprising tin oxide;
   - and any combination of the above;
c. printing the third structure made of electrochromic material on the second transparent conductive structure obtained in step (b);
d. depositing a fourth dielectric structure, preferably a layer of dielectric resin, on the face a of the material obtained after step (c) leaving at least one area exposed corresponding to the deposit area of the sample of the third structure;
e. printing a series of conductive tracks on the structure made of dielectric material deposited in step (d), such that the ends of the tracks coincide with the inner ends of the antenna and with the contact of the auxiliary electrode deposited in step (a);
f. depositing a new structure made of dielectric material, defining the working areas of the electrodes, the contacts for the diode and other possible electronic or measurement components, and the area wherein the sample to be analysed will be confined;
g. assembling a diode on the conductive tracks in the area suitable to do so by means of silver paste or an anisotropic conductive adhesive; and
h. adhering a transparent structure comprising chitosan and an oxidase enzyme in the deposit area of the sample of the fourth structure free from dielectric structure.

In a preferred embodiment of the method for obtaining the device of the present invention described, it comprises
additionally a step (c') after step (c) of printing a layer on the transducer of the auxiliary electrode selected from a transparent conductive structure or an electrochromic structure.

Another aspect of the present invention relates to another alternative method for obtaining the device of the present invention, characterised in that it comprises the following steps
a. printing on the face a of the substrate at least two second transparent conductive second structures by using a transparent conductor in order to function as transducers, one for the working electrode and the other for the auxiliary electrode, preferably selected from
   µ at least one layer of PEDOT: PSS;
   µ at least one thin layer with a size between 3 nm and 10 nm of a material selected from Au, Pt, graphene, carbon nanotubes, fullerenes;
   µ at least one layer comprising tin oxide;
   µ and any combination of the above;
b. printing, preferably by means of screen printing, a second conductive structure on the printing performed in step (a) by using conductive silver paste to form the conductive tracks constituting the spiral structure of the antenna and the conductive tracks of the working and auxiliary electrodes;
c. printing the electrochromic material on the second conductive structure obtained in step (b);
d. printing a series of conductive tracks on the second face b of the substrate, such that the ends of the tracks coincide with the areas of pathways and make contact with the ends of the antenna and with the contact of the auxiliary electrode;
e. depositing a fifth dielectric structure, preferably a layer of dielectric resin, on the face a of the material obtained after step (d) leaving at least one area exposed corresponding to the deposit area of the sample of fourth structure;
f. assembling a diode on the conductive tracks of face b of the substrate obtained in step (d), in the area suitable to do so by means of silver paste or an anisotropic conductive adhesive; and
g. adhering a transparent structure comprising chitosan and an oxidase enzyme in the deposit area of the sample of the fourth structure free from dielectric structure;

In a preferred embodiment of the method for obtaining the device of the present invention described, it comprises additionally a step (c') after step (c) of printing a layer on the transducer of the auxiliary electrode selected from a transparent conductive structure or an electrochromic structure.

In the present invention, "to print or printing" are understood as any layer or structure which can be manufactured by means of techniques such as screen printing, ink-jet printing, or offset printing, or by means of a combination of techniques from the aforementioned fields, as well as microfabrication.

Another aspect of the invention is a patch or label comprising the device of the present invention.

Another aspect of the present invention relates to the use of the patch or label of the present invention for the qualitative and/or quantitative detection of an analyte in a liquid sample.

The liquid sample can be selected from a biological fluid or a food. In a preferred embodiment, the biological fluid is sweat.

The analyte to be determined can be selected from glucose, lactose, maltose, uric acid or ethanol, among others, more preferably the analyte which is determined is glucose.

Another aspect of the invention relates to a system characterised in that it comprises:
a. the device of the present invention; and
b. emission means for a signal in a radio frequency between 10.56 MHz and 16.56 MHz configured to restart the measurement of the device until the non-reading state or state of absence of analyte.

In a more preferred embodiment of the system of the present invention, the signal emission means are selected from mobile devices with Near-Field Communication (NFC) capability, Near-Field Communication (NFC) readers, smart phone apparatuses or smart watches.

Another aspect of the present invention relates to a method for the qualitative and/or quantitative detection of an analyte in a liquid sample comprising:
(i) putting the liquid sample in contact with the transparent structure configured to deposit the sample on it; and
(ii) detection of the analyte by means of the colour change.

A preferred embodiment of the method of the present invention comprises an additional step, after step (ii), comprising step (iii) of restarting the device by means of sending a signal at a certain radio frequency between 10.56 MHz and 16.56 MHz. In this manner, we prepare the device for the detection of another sample according to steps (i) and (ii).

Throughout the description and in the claims, the word "comprises" and its variants are not intended to exclude other technical characteristics, additives, components or steps. For those skilled in the art, other objects, advantages and features of the invention may be deduced from both the description and the practical use of the invention. The following examples and drawings are provided by way of illustration, and are not meant to limit the present invention.

### BRIEF DESCRIPTION OF THE FIGURES

**FIG. 1** shows a diagram of the assembly of one of the embodiments and of the portions of the device of the present invention.
**FIG. 2** shows a diagram of the assembly of another of the embodiments and of the portions of the device of the present invention.
**FIG. 3** shows a diagram of the assembly of another of the embodiments and of the portions of the device of the present invention.
**FIG. 4** shows a diagram of the biosensor device of the present invention.
**FIG. 5** shows cyclic voltammograms obtained in supporting electrolyte for the different types of electrodes.
**FIG. 6** shows cyclic voltammetry in supporting electrolyte and derivative voltabsorptogram for SiO₂-ATO electrodes.
**FIG. 7** shows cyclic voltammetry in supporting electrolyte and derivative voltabsorptogram for ITO electrodes.
**FIG. 8** shows the evolution of the absorbance when alternating the potential from -0.1 to +0.4 V vs Ag/Ag⁺ in 60s intervals in a 0.25 mM H₂O₂ solution in supporting electrolyte.
**FIG. 9** shows the temporal evolution of the absorbance spectra for an ITO/PB electrode in a 0.25mM H₂O₂ solution in supporting electrolyte.
**FIG. 10** shows the temporal evolution of the absorbance measured at 700nm for different concentrations of hydrogen peroxide in an ITO electrode.
**FIG. 11** shows a representation of the absorbance measured at 700nm at a fixed time of 100s for an ITO/PB electrode (triangles) and another SiO₂-ATO/PB electrode (circles).
**FIG. 12** shows amperometric calibration curves of the glucose biosensor obtained in an ITO/PB electrode (triangles) and another SiO₂-ATO/PB electrode (circles).
**FIG. 13** shows spectroscopic calibration curves of the glucose biosensor obtained in an ITO/PB electrode (triangles) and another SiO₂-ATO/PB electrode (circles).

### EXAMPLES

The invention, is illustrated below by means of assays carried out by the inventors which reveal the effectiveness of the product of the invention.

### Example 1.

Obtaining the glucose biosensor device comprising PEDOT:PSS as a transparent structure located on the substrate.

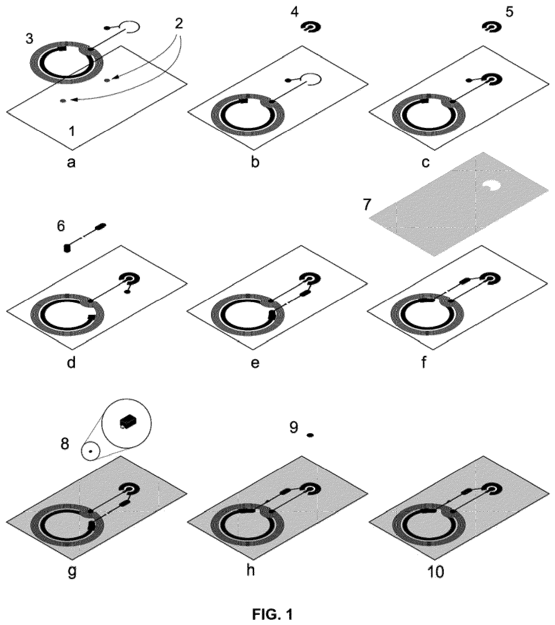

As seen in Figure 1:
(a) First, in a transparent PET substrate with a thickness (1) between 30 µm -100 µm, a series of through holes (2) are made in certain areas, corresponding to the pathways which will serve to facilitate the electrical contact between the two faces of the substrate. The pathways are made by means of a CO₂ laser. On said substrate, the main structure of the antenna and the conductive tracks (3) for the working and auxiliary electrodes are printed by means of screen printing. In our case, conductive silver paste is used and the printing is by means of screen printing.
(b) Next, the transducers for the working (sensor) and auxiliary (4) electrodes are printed. The material of these transducers will be a transparent conductor, preferably PEDOT:PSS.
(c) Next, the electrochromic material (5) comprising SiO₂-ATO or ITO is printed, at least on the central transducer and, additionally, also on the auxiliary electrode.
(d) The next step consists of printing a series of conductive tracks on the opposite face of the substrate, such that the ends of the tracks (6) coincide with the areas of pathways and make contact with the ends of the antenna and with the contact of the auxiliary electrode, which is located on the front face;
(e) having done this, the substrate is turned again;
(f) and the antenna and the conductive tracks are protected with a structure made of dielectric material (7), Loctite ^{©} EDAG-PF 455BC. This structure, in turn, defines the working areas of the electrodes and the area wherein the sample to be analysed will be confined. The reason to print the dielectric after having completed the printing of the conductive material on both faces is to prevent blocking the areas of pathways in case the first printing (a) was not perfect. In this case, a photocurable resin is used.
(g) The diode (8) is then assembled on the front face. In order to fasten it we will use a silver paste or an anisotropic conductive adhesive. In our case, we preferably use Loctite 3880 conductive silver paste for the assembly of discrete components.
   In particular, a Schottky diode from the Panasonic brand, reference DB2S20500L. The dimensions thereof are 0.8 mm x 1.6 mm x 0.6 mm (x, y, z).
(h) Finally, a transparent structure (9) comprising chitosan and an oxidase enzyme is adhered to the deposit area of the sample and which is free of dielectric structure and defined by it. Finally, the biosensor device (10) is obtained.

Although the sample is placed on the face of the sensor, the fact of having transparent electrodes on a transparent substrate makes the colour change also be able to be read through the rear face of the device.

### Example 2.

Obtaining the glucose biosensor device comprising ITO as a transparent structure located on the face a of the substrate.

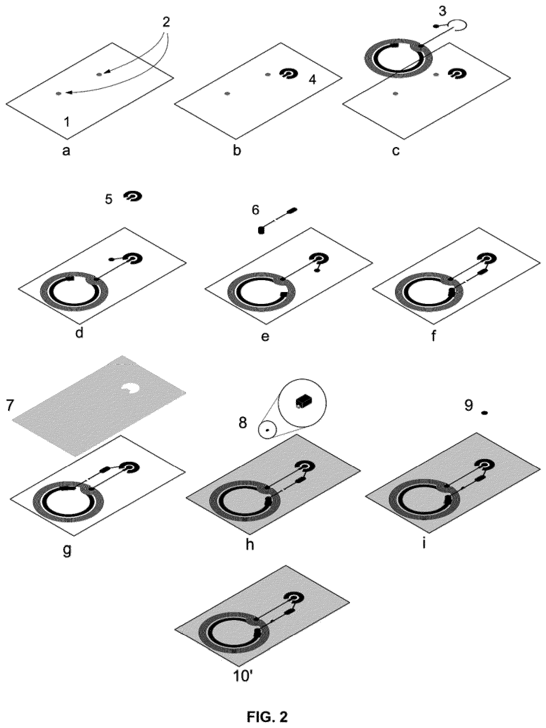

As seen in Figure 2:
(a) First, in a transparent ITO-PET substrate with a total thickness (1) between 30 µm - 100 µm, a series of through holes (2) are made in certain areas, corresponding to the pathways which will serve to facilitate the electrical contact between the two faces of the substrate. The pathways are made by means of a CO₂ laser.
(b) Next, an etching must be done to define the areas of the working and auxiliary electrodes, in the ITO. This etching is performed as follows: first, the ITO-PET sheet is pre-cut and a series of recording marks are made to facilitate the alignment of the subsequent printing steps. Also, some marks are made in order to align a vinyl mask, which defines the areas which must be etched. Once the areas of the electrodes are protected, the substrate is treated in a bath of diluted aqua regia (HCl-HNO₃) which removes the ITO from areas where it is not needed. Subsequently, the substrate is rinsed with deionised water, and dried. The process continues.
   If the ITO-coated PET sheet is protected by means of plastic, the process can be simplified as follows: By means of a cutting tool, the areas of the electrodes are profiled and recording marks are made on the substrate. Next, the protector is removed from the areas from which the ITO is to be removed, which is etched in a diluted aqua regia bath as described in the previous paragraph. After removing the ITO, the substrate is rinsed with deionised water, and dried. The remaining protector is removed, leaving the transducers of the ITO electrodes uncovered, and the process continues.
(c) On said substrate, the main structure of the antenna and the conductive tracks for the working and auxiliary electrodes (3) are printed by means of screen printing. In our case, conductive silver paste is used and the printing is by means of screen printing. The process continues with steps (d)-(h) of example 1, until the biosensor device 10' is obtained.

### Example 3

Obtaining the glucose biosensor device comprising PEDOT:PSS as a transparent conductive layer located on the face a of the substrate.

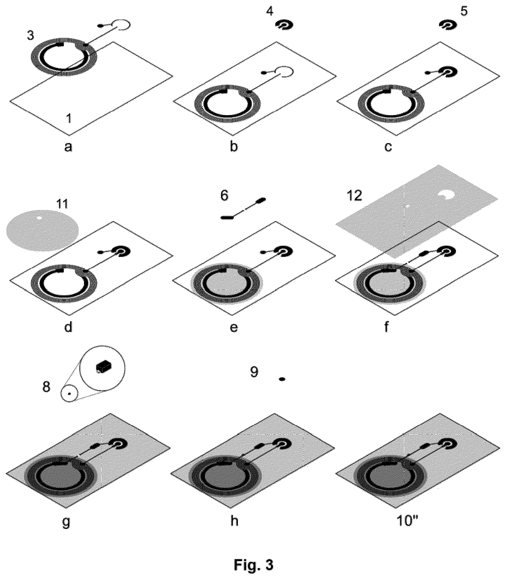

As seen in Figure 3:
(a) First, on a transparent PET substrate with a thickness (1) between 30 µm -100 µm, the main structure of the antenna and the conductive tracks for the working and auxiliary electrodes (2) are printed by means of screen printing. In our case, conductive silver paste (Loctite EDAG PM-406V1) is used and the printing is by means of screen printing.
(b) Next, the transducers for the working (sensor) and auxiliary (3) electrodes are printed. The material of these transducers will be a transparent conductor, in this case it is PEDOT:PSS.
(c) Next, the electrochromic structure comprising SiO₂-ATO (5) is printed on the transducers.
(d) Subsequently, a structure made of dielectric material is deposited such that it partially protects the antenna, leaving at least the inner end thereof (11) exposed.
(e) The next step consists of printing a series of conductive tracks on the structure made of dielectric material deposited in the previous step, such that the ends of the tracks (6) coincide with the inner ends of the antenna and with the contact of the auxiliary electrode deposited in step (a);
(f) Next, a new structure made of dielectric material (12) is deposited, in our case, Loctite^{©} EDAG-PF 455BC). This structure, in turn, defines the working areas of the electrodes, the contacts for the diode and other possible electronic or measurement components, and the area wherein the sample to be analysed will be confined. The aim of this structure is to protect the conductive tracks from degradation by the environment, as well as to prevent possible measurement errors caused by spillage of the sample beyond the area of the electrochromic and auxiliary electrodes.
(g) Finally, a diode (8) is assembled, in our case it is the reference DB2S20500L, from Panasonic.
(h) Finally, a transparent structure (9) comprising chitosan and an oxidase enzyme is adhered to the deposit area of the sample and which is free of dielectric structure and defined by it. The finished biosensor device (10") is shown.

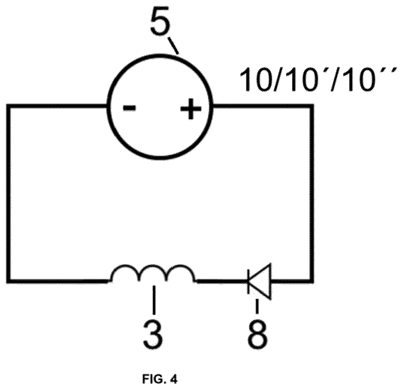

The sensor device of the present invention according to examples 1 to 3 is shown in a schematic view in Figure 4, and they show a colour change in the presence of the analyte to be determined. The concentration of said analyte is calculated starting from the speed of the colour change and the intensity thereof.

### Example 4

Electrochemical performance of the electrodes the electrochromic structure of which comprises SiO₂-ATO or ITO and the comparison thereof with the electrodes already present in the state of the art comprising in the electrochromic structure thereof a commercial graphite paste (DropSens electrodes with reference DRP-710), or with commercial modified screen-printed carbon electrodes (screen-printed electrodes using Gwent graphite paste with reference C2070424P2).

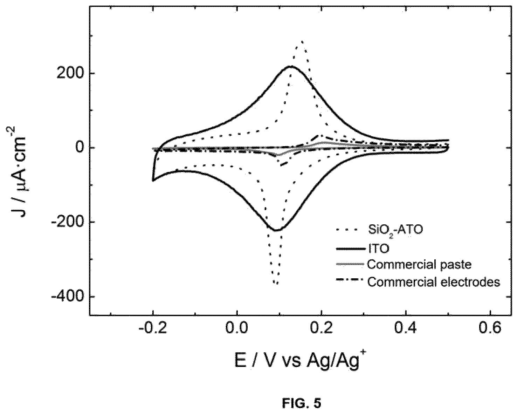

Figure 5 shows the cyclic voltammograms recorded in the supporting electrolyte for all the electrodes (SiO₂-ATO, ITO, commercial graphite paste, commercial modified screen-printed carbon electrodes). The most striking difference in the voltammetry of the different pastes modified with Prussian Blue is the much higher current observed for the blue pastes presented herein, in comparison to those obtained from commercial materials.

The blue electrodes show superior electrochemical behaviour based on the peak-to-peak separation thereof compared to commercial graphite-based materials (Table 1)

**Table 1: Data obtained from the voltammetry experiments**

| | ΔEp/mV | Jp,c/µA·cm⁻² | Charge density/mC·cm⁻² |
|---|---|---|---|
| SiO₂-A TO/Prussian Blue | 60±2 | 369±1 | 8.9±0.1 |
| ITO/Prussian Blue | 31±2 | 243±1 | 11.0±0.1 |
| DS/Prussian Blue | 82±2 | 42±1 | 0.6±0.1 |
| Gwent/Prussian Blue | 87±2 | 73±1 | 1.4±0.3 |

This is due to a better contact between Prussian Blue and the conductive material of the particles. Furthermore, the nanoparticles modified with ITO show the smallest peak-to-peak separation, ca. 30 mV to 5 mVs⁻¹, probably due to the better conductivity offered by ITO nanoparticles compared to the SiO₂-ATO microparticles. However, the much higher load of Prussian Blue than the state of the art, which arises from the available massive surface area of the nanoparticles, combined with the thickness of the layer printed on the screen, produces much wider peaks in comparison with the other materials.

### Example 5.

Spectroelectrochemical performance of the electrodes the electrochromic structure of which comprises SiO₂-ATO or ITO and the comparison thereof with the electrodes already present in the state of the art comprising in the electrochromic structure thereof a commercial graphite paste, or with commercial modified screen-printed carbon electrodes, in the detection of H₂O₂, simulating the conditions of a biosensor based on the reaction of an oxidase wherein said hydrogen peroxide is produced and the optical quantification thereof.

The black colour of the graphite in the commercial electrodes modified with Prussian Blue prevents the observation of any spectroelectrochemical change.

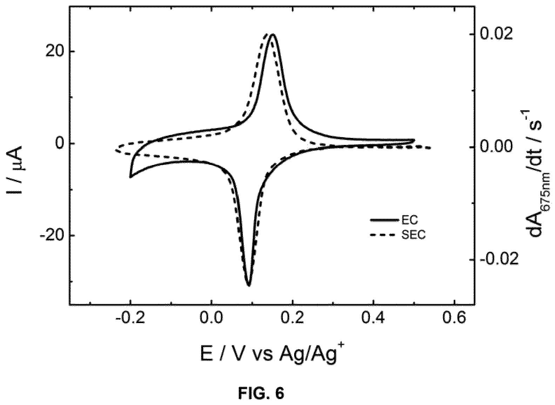

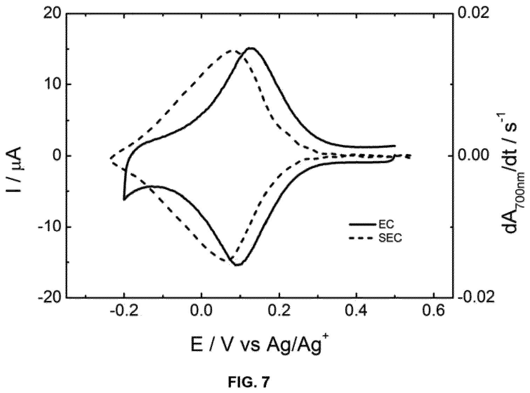

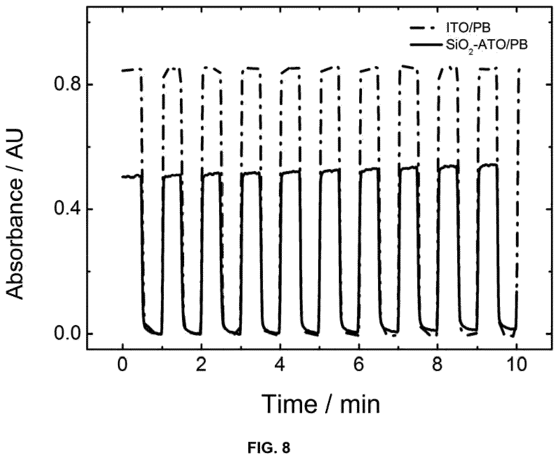

In contrast, the cyclic voltammograms, with a sweep speed of 5 mVs⁻¹, of the electrodes the electrochromic structure of which comprises SiO₂-ATO (Figure 6) or ITO (Figure 7) presented herein, the colour change associated with the redox process of Prussian Blue is clearly observed. Furthermore, it is observed together with the derivative of the respective voltabsorptograms thereof with an evolution of the absorbance when alternating the potential -0.1 to +0.4 V vs Ag/Ag+ in intervals of 60s in a 0.25 mM H₂O₂ solution in the supporting electrolyte. Figure 8 shows the change in the absorbance as reducing and oxidising potentials are applied. The data shows that the electrodes the electrochromic structure of which comprises ITO show a greater contrast between the oxidised and reduced states than the electrodes the electrochromic structure of which comprises SiO₂-ATO/Prussian Blue.

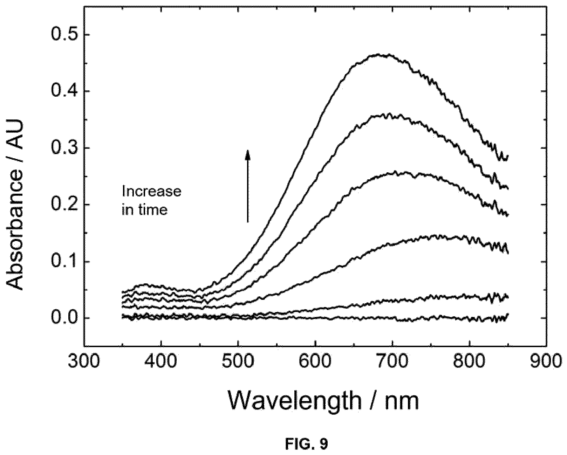

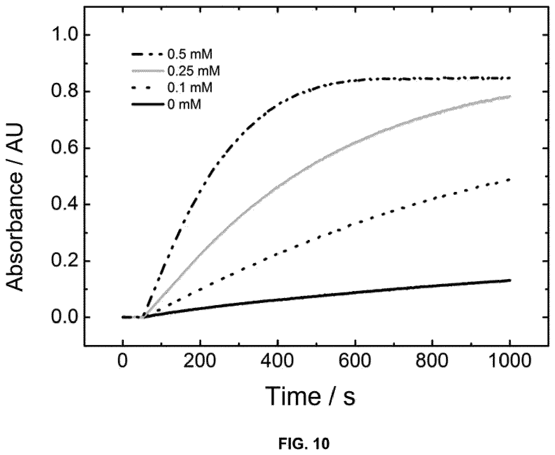

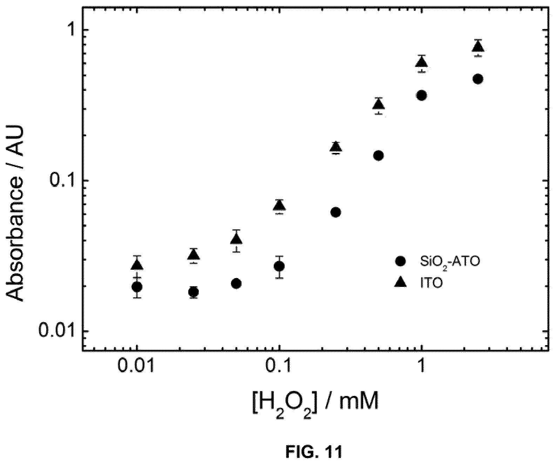

For the optical quantification of the hydrogen peroxide the measurement was performed as follows. First, Prussian Blue was reduced to Prussian White by means of applying a potential of -0.1 V vs. Ag/Ag+. Once a stable background colourimetric signal was observed, after approximately 60 seconds of polarisation of the electrode, the potentiostat was turned off, leaving the electrochemical cell in an open circuit. Then, the hydrogen peroxide present in the solution chemically oxidised the Prussian White back to Prussian Blue, and the corresponding colour change was monitored by means of UV-Vis reflectance (Fig. 9). The maximum colour contrast depends on the colour of the underlying conductive particles, which were white (SiO₂-ATO) or pale yellow (ITO), respectively. It was found that these were 675 nm for SiO₂-ATO/Prussian Blue electrodes and 700 nm for ITO/Prussian Blue. Figure 10 shows diagrams of absorbance as a function of the time at these wavelengths, as a means of monitoring the reduction of hydrogen peroxide in the PW/Prussian Blue surface. As the data shows, the higher the concentration of H₂O₂, the faster the electrode fully recovers the blue colour thereof. Figure 11 shows the relationship between the peroxide concentration and the reflectance measured at 700 nm, 100 seconds after the depolarisation of the electrode. Similar to the coulometric sensors, the sensitivity and detection limit of which can be adjusted by choosing a suitable integration time, here the performance of the sensor can be adjusted to the concentration of the sample simply by adjusting the integration time of the spectrophotometer and/or the sampling time after the depolarisation of the electrode (in our case 300 ms and 100 s, respectively). This provides control over the sensitivity, the linear range, and the detection limit of the method (see Table 2).

**Table 2: Spectroscopic determination of H₂O₂ at different sampling times.**

| | SiO₂-ATO | | | ITO | | |
|---|---|---|---|---|---|---|
| | Sensitivity/AU·mM⁻¹ | Dynamic range/mM | LD/µM | Sensitivity/AU·mM^{- 1} | Dynamic Range/mM | LD/µM |
| t = 30s | 0.101 | 0.05 - 5 | 12±10 | 0.206 | 0.025 - 5 | 8±7 |
| t = 100s | 0.318 | 0.05 - 2.5 | 4±3 | 0.591 | 0.025 - 2.5 | 3±2 |
| t = 200s | 0.729 | 0.05 - 1 | 2±1 | 1.084 | 0.025 - 1 | 2±1 |
| t = 300s | 0.958 | 0.025 - 0.5 | 1±1 | 1.335 | 0.025 - 1 | 1±1 |

As the table shows, the analytical parameters of the sensor can be controlled through the sampling time of the optical measurements. Increasing the sampling time enables the sensitivity of the measurement to be improved, and enables lower analyte concentrations to be detected. Moreover, the dynamic range of the sensor is narrowed, since the photodetector is saturated at lower concentrations as a direct consequence of the longer sampling time.

### Example 6

The devices of examples 1 and 2 are used for the determination of glucose in sweat. The enzymatic oxidation of the glucose produces hydrogen peroxide which, in the electrodes modified with SiO₂-ATO and Prussian Blue, enables the quantification of the glucose electrochemically and spectroscopically.

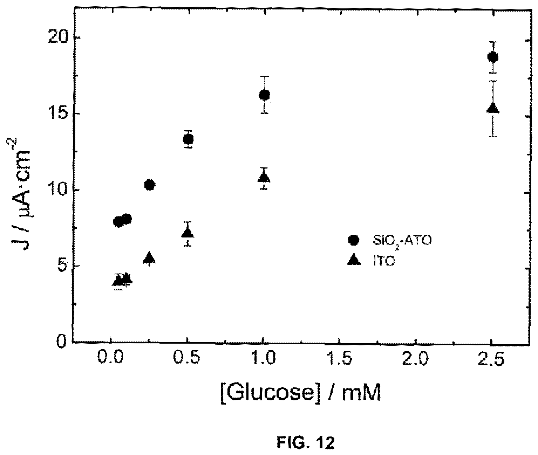

Figure 12 shows the Michaelis-Menten amperometric plots for the glucose by using the two electrochromic devices manufactured as indicated in examples 1 and 2. The dynamic range of both biosensors is comprised between 0.1 mM and 1 mM of glucose, and reaches a saturation greater than 2.5 mM. The analytical performance of our blue biosensors in terms of detection limit and sensitivity, ca. 60 µM and 8·10⁻³ A·cm⁻²·M⁻¹ (see Table 3) is comparable to that of other reported amperometric biosensors.

**Table 3: Amperometric and spectroscopic determination of the glucose for the two types of electrode**

| | Amperometry | | Spectroscopy | |
|---|---|---|---|---|
| | LD/µM | Sensitivity/A·cm⁻²·M⁻¹ | LD/µM | Sensitivity/AU·mM⁻¹ |
| SiO₂-A TO/Prussian Blue | 16±4 | 8.8·10⁻³ | 9±1 | 0.21 |
| ITO/Prussian Blue | 34±6 | 7.4·10⁻³ | 6±1 | 0.19 |

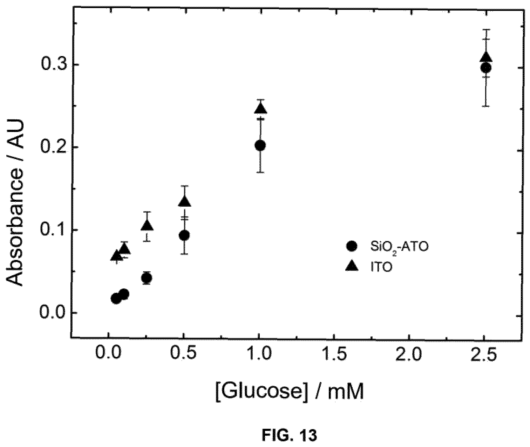

Figure 13 shows the spectroscopic calibration plots of the glucose biosensor for the two different devices. As seen, no substantial differences are observed when the amperometric or spectroscopic detection method is used, since a similar dynamic range is reached in all cases, reaching a plateau in both cases at concentrations above 2.5 mM. A slight gain in the detection limit is achieved since it decreases to approximately 15 µM probably due to the effect of the high background currents present in amperometric detection which disappear when a spectroscopic detection method is used, but, ultimately, the yields of both types of electrochromic electrodes are comparable, regardless of the device used.

## Claims

1. A wireless and portable biosensor device for displaying a colour change from a first colour to a second colour, wherein said second colour indicates the presence of an analyte in a liquid sample, and the first colour indicates the absence of the analyte or a non-reading state, the device being **characterised in that** it comprises a stratified structure comprising:
• a transparent substrate comprising a face a and a face b, optionally the substrate comprises at least two holes connecting the two faces, a and b, of the substrate;
• a conductive structure configured as the main structure of an antenna and at least one conductive track of the working and auxiliary electrodes, comprising conductive silver paste;
• at least two transparent conductive structures configured to function at least as two transducers, one for the working electrode and the other for the auxiliary electrode;
• at least one electrochromic structure, configured to function at least as a working electrode;
• a transparent structure configured to deposit the sample on it and comprising
▪ chitosan; and
▪ an oxidase enzyme.
• a main dielectric structure which is configured to insulate the conductive tracks from the conductive structure;
• optionally a second dielectric structure located on the conductive structure and/or on the electrochromic one, leaving at least one area of said electrochromic layer exposed, and wherein said dielectric structure layer comprises at least two holes;
• at least one conductive track configured to connect with the conductive structure in order to close the circuit; and
• a diode;
wherein the device functions in direct current;
wherein the antenna is configured so that, with a radio frequency between 10.56 MHz and 16.56 MHz, the measurement of the device is restarted until the non-reading state or state of absence of analyte in the sample; and
wherein the device is configured to be reset to the non-reading state or state of absence of analyte thereof when receiving a signal of a certain radio frequency between 10.56 MHz and 16.56 MHz.

2. The device according to claim 1, wherein the conductive structure, comprising conductive silver paste, is located directly on the face a of the substrate.

3. The device according to claim 2, wherein the transparent conductive structure is located on the conductive structure located on the face a of the substrate.

4. The device according to claim 3, the device being **characterised in that** it comprises a stratified structure comprising:
• a transparent substrate comprising a face a and a face b, and wherein the substrate comprises at least two holes connecting the two faces, a and b, of the substrate;
• a first layer of conductive structure configured as the main structure of an antenna and conductive tracks of the working and auxiliary electrodes, comprising conductive silver paste, and located on at least one portion of the face a of the substrate;
• at least one second layer of two transparent conductive structures, located on the first layer of conductive structure, and configured to function at least as two transducers, one for the working electrode and the other for the auxiliary electrode;
• at least one third layer of electrochromic structure, located on at least one area of the second layer of conductive structure, and configured to function at least as a working electrode;
• a fourth layer of transparent structure adhered on the third layer of electrochromic structure, and configured to deposit the sample to be determined on it and comprising:
▪ chitosan; and
▪ an oxidase enzyme.
• a fifth layer of main dielectric structure located on the previous layers leaving at least one area exposed corresponding to the deposit area of the sample of the fourth layer, and which is configured to insulate the conductive tracks of the main structure of the first layer;
• a sixth layer of a conductive track comprising conductive silver paste, and located on the face b of the substrate and configured to connect, by means of the holes in the substrate, with the conductive structure of the first layer and to close the circuit; and
• a diode on the sixth layer.

5. The device according to claim 1, wherein the transparent conductive structures, configured to function as two transducers, one for the working electrode and the other for the auxiliary electrode, are located directly on the face a of the substrate.

6. The device according to claim 5, wherein the conductive structure, comprising conductive silver paste, is located on the transparent conductive structure.

7. The device according to claim 6, the device being **characterised in that** it comprises a stratified structure comprising:
• a transparent substrate comprising a face a and a face b, and wherein the substrate comprises at least two holes connecting the two faces, a and b, of the substrate;
• at least one first layer of transparent conductive structures located on the face a of the substrate and configured to function as two transducers, one for the working electrode and the other for the auxiliary electrode;
• a second layer of conductive structure, configured as the main structure of an antenna and conductive tracks of the working and auxiliary electrodes, and comprising conductive silver paste, and in contact with at least one portion of the first layer of transparent conductive structure;
• at least one third layer of electrochromic structure, located on at least one surface of the second layer of conductive structure, configured to function at least as a working electrode;
• a fourth layer of transparent structure adhered on the third layer of electrochromic structure configured to deposit the sample on it and comprising
▪ chitosan; and
▪ an oxidase enzyme.
• a fifth layer of main dielectric structure located on the previous layers leaving at least one area or surface exposed or uncovered corresponding to the deposit area of the sample described in the fourth layer, and which is configured to insulate the conductive tracks of the second layer;
• a sixth layer of a conductive track comprising conductive silver paste, and located on the face b of the substrate and configured to connect, by means of the holes in the substrate, with the conductive structure of the first layer and to close the circuit; and;
• a diode on the sixth layer.

8. The device according to claim 6, the device being **characterised in that** it comprises a stratified structure comprising:
• a transparent substrate comprising a face a and a face b;
• at least one first layer of transparent conductive structures located on one of the faces of the substrate and configured to function as two transducers, one for the working electrode and the other for the auxiliary electrode;
• a second layer of conductive structure, configured as the main structure of an antenna and conductive tracks of the working and auxiliary electrodes, and comprising conductive silver paste, and contact with at least one portion of the first layer of transparent conductive structures;
• a third layer of dielectric structure configured to insulate the conductive tracks of the second layer;
• at least one fourth layer of electrochromic structure, located on at least one area of the second layer of conductive structure, configured to function at least as a working electrode;
• a fifth layer of a second dielectric structure located on the fourth layer of electrochromic structure leaving at least one area exposed or uncovered, corresponding to the deposit area of the sample, and wherein said dielectric structure comprises at least two holes;
• a sixth layer of transparent structure located, in the area exposed or uncovered by the fifth layer, on the fourth layer of electrochromic structure, configured to deposit the sample on it and comprising:
▪ chitosan; and
▪ an oxidase enzyme.
• a seventh layer of a conductive track, configured to connect with the second layer of conductive structure through the holes in the fifth layer of dielectric structure and to close the circuit; and
• a diode on the seventh layer of conductive structure.

9. The device according to any of claims 1 to 8, wherein the diode is a Schottky diode and has dimensions between 0.8 mm and 1.6 mm.

10. The device according to any of claims 1 to 9, wherein the oxidase enzyme is selected from glucose oxidase, lactose oxidase, maltose oxidase, urate oxidase or ethanol oxidase.

11. The device according to any one of claims 1 to 10, wherein the oxidase enzyme is glucose oxidase.

12. The device according to claim 11, wherein it additionally comprises mutarotase enzyme.

13. The device according to any of claims 1 to 12, wherein the device comprises an additional structure on the transducer of the auxiliary electrode configured to function as an auxiliary electrode.

14. The device according to claim 13, wherein the additional structure is selected from a transparent conductive structure or an electrochromic structure.

15. The device according to claim 14, wherein the additional structure is an electrochromic structure.

16. The device according to any of claims 1 to 15, wherein each electrochromic structure comprises
a. microparticles, selected from
i. mixed indium and tin oxide;
ii. of the core-shell type wherein the core is SiO₂ and wherein the shell is a mixed antimony and tin oxide; and
iii. any combination of the above;
b. an ink comprising
i. pigment, preferably Prussian Blue;
ii. a binding resin, preferably a Viton solution; and
wherein the microparticle:ink ratio is 2.5:1;
and wherein the microparticles and the paint form a homogeneous particulate mixture with a particle size of said mixture between 3 µm and 12 µm.

17. A method for obtaining the device of claim 4, **characterised in that** it comprises the following steps
a. making holes in a transparent substrate comprising a face a and a face b by means of a technique selected from CO₂ laser, mechanical punching and a combination of the above, such that they connect the two faces of the substrate;
b. printing a conductive structure on the face a of the substrate using conductive silver paste in order to form the spiral structure of the antenna and the conductive tracks of the working and auxiliary electrodes, making at least one printed area coincide with the holes in the step (a);
c. printing at least two transparent conductive structures on the printing performed in step (b) by using a transparent conductor, to function as transducers, one for the working electrode and the other for the auxiliary electrode;
d. printing an electrochromic structure on a portion of the conductive structure obtained in step (c);
e. printing a conductive track on the face b of the substrate, such that the ends of the tracks coincide with the areas of the holes and make contact with the ends of the antenna and with the transducer of the auxiliary electrode;
f. depositing a dielectric structure on the face a of the material obtained after step (d) leaving at least one area of the electrochromic structure exposed corresponding to the deposit area of the sample;
g. depositing a diode on the conductive track of the face b of the substrate obtained in step (e), between one of the ends of the antenna and one of the electrodes making up the sensor, by means of silver paste or an anisotropic conductive adhesive; and
h. adhering a transparent structure comprising chitosan and an oxidase enzyme in the deposit area of the sample which remained exposed after depositing the dielectric structure in step (f).

18. The method for obtaining the device according to claim 17, wherein it comprises additionally a step (c') after step (c) of printing a layer on the transducer of the auxiliary electrode configured to function as an auxiliary electrode and selected from a transparent conductive structure or an electrochromic structure.

19. The method for obtaining the device of claim 7, **characterised in that** it comprises the following steps
a. making holes in a transparent substrate comprising a face a and a face b by means of a technique selected from CO₂ laser, mechanical punching and a combination of the above, such that they connect the two faces of the substrate;
b. printing on the face a of the substrate at least two transparent conductive structures by using a transparent conductor in order to function as transducers, one for the working electrode and the other for the auxiliary electrode,
c. printing a second conductive structure on the printing performed in step (b) by using conductive silver paste in order to form the conductive tracks constituting the spiral structure of the antenna and the conductive tracks of the working electrodes;
d. printing an electrochromic structure on the second conductive structure obtained in step (c);
e. printing a conductive track on the face b of the substrate, such that the ends of the tracks coincide with the areas of the holes and make contact with the ends of the antenna;
f. depositing a dielectric structure on the face a of the material obtained after step (e) leaving at least one area exposed corresponding to the deposit area of the sample of the electrochromic structure and
g. assembling a diode on the conductive tracks of the face b of the substrate obtained in step (e), between one of the ends of the antenna and one of the electrodes making up the sensor, by means of silver paste or an anisotropic conductive adhesive; and
h. adhering a transparent structure comprising chitosan and an oxidase enzyme in the deposit area of the sample of the structure free from dielectric structure of step (f).

20. The method for obtaining the device according to claim 19, wherein it comprises additionally a step (d') after step (d) of printing a layer on the transducer of the auxiliary electrode selected from a transparent conductive structure or an electrochromic structure.

21. The method for obtaining the device of claim 8, **characterised in that** it comprises the following steps
a. printing on the face a of the transparent substrate a conductive structure, using conductive silver paste to form the conductive tracks constituting the structure of the antenna and the conductive tracks of the working and auxiliary electrodes;
b. printing on the conductive structure obtained in step (a), at least two second transparent conductive structures by using a transparent conductor to function as transducers, one for the working electrode and the other for the auxiliary electrode;
c. printing an electrochromic structure on the transparent structure obtained in step (b);
d. depositing a dielectric structure on the face a of the material obtained after step (c) leaving at least one area exposed corresponding to the deposit area of the sample;
e. printing at least one conductive track on the dielectric structure of step (d), such that the ends of the tracks coincide with the inner ends of the antenna and with the contact of the auxiliary electrode;
f. depositing another dielectric structure, leaving the working areas of the electrodes, the contacts for the diode, and the area of the sample to be analysed;
g. assembling a diode on the conductive tracks by means of silver paste or an anisotropic conductive adhesive; and
h. adhering a transparent structure comprising chitosan and an oxidase enzyme in the deposit area of the sample of the structure free from dielectric structure of step (f).

22. The method for obtaining the device according to claim 21, wherein it comprises additionally a step (c') after step (c) of printing a layer on the transducer of the auxiliary electrode selected from a transparent conductive structure or an electrochromic structure.

23. A patch or label comprising the device according to any of claims 1 to 16.

24. A use of the patch according to claim 23 or the device according to any of claims 1 to 16, for the qualitative and/or quantitative detection of an analyte in a liquid sample.

25. The use according to claim 24, wherein the liquid sample is selected from a biological fluid or a food.

26. The use according to claim 25, wherein the biological fluid is sweat.

27. The use according to any of claims 23 to 26, wherein the analyte which is determined is glucose.

28. A system **characterised in that** it comprises
a. the portable and wireless biosensor device described in any of claims 1 to 16; and
b. emission means for a signal in a radio frequency between 10.56 MHz and 16.56 MHz configured to restart the measurement of the device until the non-reading state or state of absence of analyte.

29. The system according to claim 28, wherein the emission means of the radio frequency signal are selected from mobile devices with Near-Field Communication capability, Near-Field Communication readers, smart phone apparatuses, smart watches.

30. A method for the qualitative and/or quantitative detection of an analyte in a liquid sample comprising:
(i) putting the liquid sample and the transparent structure of the device described in any of claims 1 to 16 in contact; and
(ii) detection of the analyte by means of the colour change.

31. The method according to claim 30, wherein it comprises a step after step (ii) of restarting the device by means of sending a signal at a certain radio frequency between 10.56 MHz and 16.56 MHz, for the detection of another sample according to steps (i) and (ii).
